Europäisches Patentamt

European Patent Office

Office européen des brevets

(11) Veröffentlichungsnummer: **0 228 537**
**A2**

(12) **EUROPÄISCHE PATENTANMELDUNG**

(21) Anmeldenummer: 86115368.2

(51) Int. Cl.⁴: **A61N 1/42 , A61N 5/06**

(22) Anmeldetag: 06.11.86

(30) Priorität: **19.11.85 DE 8532628 U**

(43) Veröffentlichungstag der Anmeldung:
**15.07.87 Patentblatt 87/29**

(84) Benannte Vertragsstaaten:
**AT BE CH DE ES FR GB GR IT LI NL SE**

(71) Anmelder: **elec System Vertriebsgesellschaft
für medizinische Geräte mbH
Am Gemeindezentrum
D-6209 Heidenrod-Kemel(DE)**

(72) Erfinder: **Fichtner, Manfred
Am Gemeindezentrum
D-6209 Heidenrod-Kemel(DE)**

(74) Vertreter: **Weber, Dieter, Dr. et al
Dr. Dieter Weber und Klaus Seiffert
Patentanwälte Gustav-Freytag-Strasse 25
Postfach 6145
D-6200 Wiesbaden 1(DE)**

(54) **Kombiniertes Magnetfeld-Infrarot-Behandlungsgerät.**

(57) Beschrieben wird ein bioenergetisches Behandlungsgerät zur Erzeugung elektromagnetischer Felder und Wellen, durch welche bioenergetische Wirkungen im menschlichen oder tierischen Organismus hervorgerufen werden, mit einer Magnetfeldspule - (1) in einem Gehäuse bzw. einer Halterung. Um die kombinierte Anwendung von Magnetfeld-und Infrarottherapie zur gleichen Zeit und am selben Organismus zu ermöglichen, ist erfindungsgemäß vorgesehen, im Inneren der Spule (1) oder an ihrem Gehäuse mindestens einen Infrarotemitter (2) anzubringen.

Fig.1

EP 0 228 537 A2

## Kombiniertes Magnetfeld-Infrarot-Behandlungsgerät

Die Neuerung betrifft ein Behandlungsgerät zur Erzeugung elektromagnetischer Felder und Wellen, durch welche bioenergetische Wirkungen im menschlichen oder tierischen Organismus hervorgerufen werden, mit mindestens einer Magnetfeldspule in einem Gehäuse bzw. einer Halterung.

Derartige Geräte werden eingesetzt, um einen menschlichen oder tierischen Organismus vor allem einem magnetischen (Wechsel-) Feld auszusetzen, durch welche verschiedene Vorgänge in den Zellen eines Organismus in positiver Weise beeinflußt werden. Insbesondere werden derartige Geräte eingesetzt zur Behandlung von Wunden bzw. Verletzungen, Muskel-und Nervenschmerzen, Durchblutungsstörungen und dergleichen. Daneben gibt es auch die bekannten Verfahren der Mikro-oder Kurzwellentherapie und auch der Ultraschalltherapie Ebenso werden Infrarot-Bestrahlungsgeräte zur Behandlung verschiedener Krankheiten bzw. Krankheitssymptome eingesetzt.

Allerdings gibt es bisher nur wenige oder keine gezielten Versuche, die verschiedenen genannten Therapiemethoden nicht nur sukzessive und nebeneinander sondern gleichzeitig einzusetzen. Die gleichzeitige Anwendung verschiedener Therapiemethoden kann u. U. im Vergleich zu der aufeinanderfolgenden Anwendung der gleichen Methoden eine erheblich gesteigerte Wirkung zeigen.

Der Neuerung liegt daher die Aufgabe zugrunde, ein Behandlungsgerät zur Erzeugung elektromagnetischer Felder und Wellen zu - schaffen, welches die kombinierte Anwendung von Magnetfeld-und Infrarottherapie zur gleichen Zeit und am selben Organismus möglich macht.

Diese Aufgabe wird dadurch gelöst, daß bei einem Behandlungsgerät mit den eingangs erwähnten Merkmalen im Inneren der Spule oder an ihrem Gehäuse mindestens ein Infrarot-Emitter angebracht ist.

Zweckmäßiger Weise ist der Infrarot-Emitter so an der Spule bzw. im Inneren der Spule angebracht, daß seine Strahlung auf den gleichen Körperbereich fällt, in welchem auch das Magnetfeld seine Wirkung entfaltet.

Neuerungsgemäß ist dabei vorgesehen, daß der Infrarot-Emitter aus mindestens einer Infrarotlicht emittierenden Diode besteht.

Eine derartige Diode ist ein sehr kleines und handliches Bauteil, welches ohne weiteres nahezu beliebig an einer Spule angebracht werden kann. Weiterhin haben Dioden den Vorteil, daß sie elektromagnetische Strahlung bevorzugt in einem bestimmten, eng begrenzten Frequenzbereich abstrahlen.

In der bevorzugten Ausführungsform der Neuerung ist vorgesehen, daß Infrarotdioden in einer Querschnittsebene einer Magnetspule angeordnet sind.

Zu behandelnde Körperpartien werden im allgemeinen in den Bereich der Achse der Magnetspule gebracht. Sofern die Infrarotdioden in einer Querschnittsebene der Spule angebracht sind, wird der Bereich der Spulenachse auch von den Dioden sehr gleichmäßig bestrahlt. Weiterhin wird bei der Neuerung eine Ausführungsform bevorzugt, bei welcher drei Spulen mit Infrarotdioden nebeneinander angeordnet sind, wobei die Querschnittsebene der beiden äußeren Spulen mit der der mittleren Spule einen Winkel von ca. l20° einschließen.

Eine derartige geometrische Anordnung der Magnetfeldspulen hat sich als besonders günstig erwiesen, um geschlossene Magnetfeldlinien zu erzeugen, wobei bei dieser Ausführungsform gleichzeitig auch die Strahlung der in den Querschnittsebene der Spulen angebrachten Infrarotdioden auf ein gemeinsames Zentrum gerichtet ist, in welchem auch die Magnetfeldstärke relativ hohe Werte erreicht.

Bei einer anderen bevorzugten Ausführungsform der Neuerung sind Infrarotdioden auf einer zur Spulenachse konzentrischen Zylinderfläche angeordnet.

Insbesondere kann diese Zylinderfläche das Innere eines zylindrischen Spulengehäuses sein, in welches je nach Größe der Spule bzw. des Gehäuses beispielsweise verletzte Extremitäten oder auch der gesamte Körper eines Patienten eingeführt werden kann. Der Organismus ist dann in vorteilhafter Weise konzentrisch von Infrarotdioden umgeben, durch welche er mit Infrarotlicht bestrahlt wird, während gleichzeitig ein statisches oder auch zeitlich veränderliches Magnetfeld bestimmter Frequenzen und Pulsfolge im wesentlichen in Längsrichtung der Spulenachse den Organismus durchgreift.

Bevorzugt werden bei allen Ausführungsformen gemäß der Neuerung Infrarotdioden, welche Infrarotlicht im Wellenlängenbereich von ca. 900 nm ausstrahlen.

Weitere Vorteile, Merkmale und Anwendungsmöglichkeiten der vorliegenden Neuerung werden deutlich anhand der folgenden Beschreibung bevorzugter Ausführungsformen und der dazugehörigen Figuren. Es zeigen:

Figur l ein kombiniertes Magnetfeld-Infrarot-Behandlungsgerät mit drei im Winkel zueinander angeordneten Magnetspulen und

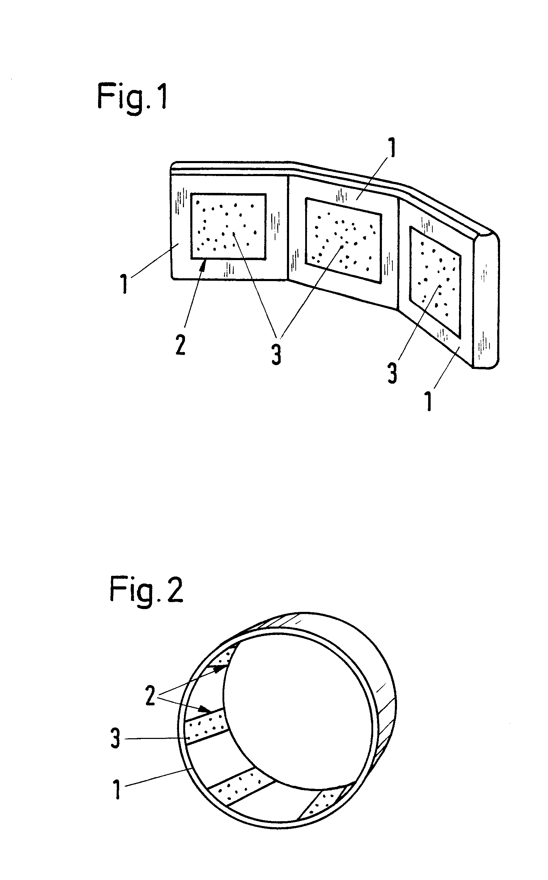

Figur 2 ein kombiniertes Magnetfeld-Infrarot-Behandlungsgerät in zylindrischer Form.

Die Ausführungsform gemäß Figur l besteht im wesentlichen aus drei Magnetspulen l, deren Öffnung im wesentlichen einen rechteckigen Querschnitt aufweist, wobei in einer Querschnittsebene der Spulenöffnung Infrarot-Emitter 2 angeordnet sind. Die Infrarot-Emitter 2 bestehen in einer bevorzugten Ausführungsform aus gleichmäßig auf den Querschnittsflächen verteilten Infrarot-Dioden 3, welche Infrarotlicht im Wellenlängenbereich von etwa 900 nm ausstrahlen.

Zur Erzielung einer besonders gerichteten bzw. punktuellen Wirkung des Infrarotlichtes können die Infrarotdioden 3 jedoch auch in einem bestimmten Schema in der Querschnittsebene angeordnet sein, wobei das von ihnen emittierte Infrarotlicht auch zusätzlich beispielsweise durch einen hinter den Dioden 3 angebrachten (Metall-) Hohlspiegel gebündelt werden kann.

Der Winkel, unter dem benachbarte Spulenelemente relativ zueinander geneigt sind, beträgt jeweils etwa 120°, so daß sich die gedachten Spulenachsen im wesentlichen in einem gemeinsamen Mittelpunkt in jeweils gleichem Abstand von den Spulenflächen schneiden.

Ein derartiges Spulensystem kann in weitgehend beliebiger Größe hergestellt und sowohl auf menschliche als auch auf tierische Organismen angewendet werden.

Bei der erwähnten Wellenlänge der Infrarotstrahlung ist deren Wirksamkeit weitgehend auf einen Oberflächenbereich bis zu einer Tiefe von ca. 5 cm beschränkt. Dabei übt das Magnetfeld einen günstigen Einfluß auf die Energiebildung in der Zelle über verbesserte $O_2$ Utilisation aus, während die Infraroteinwirkung die Zellatmungskette positiv beeinflußt.

Das Gerät dient somit zur Behandlung von Hauterkrankungen, sowie von pathologischen Erscheinungen, welche bis zu 5 cm unter der Hautoberfläche auftreten.

Je nach Wahl der Infrarotdioden 3 bzw. der Wellenlänge der von ihnen emittierten Strahlung kann man jedoch auch andere Eindringtiefen erreichen.

Eine weitere Ausführungsform des kombinierten Magnetfeld-Infrarot-Behandlungsgerätes ist in Figur 2 dargestellt. Die Magnetspule l befindet sich hier in einem als doppelwandiger Hohlzylinder ausgeführten Gehäuse. Die Infrarotdioden 3 sind hier in einer zur Spulenachse konzentrischen Zylinderfläche in Form von Infrarotemitterleisten angeordnet.

Nicht dargestellt sind die elektrischen Anschlüsse für die Magnetspule l und die Infrarotdioden 3.

Die Infrarotdioden 3 können auch auf der gesamten inneren Zylinderfläche angebracht sein.

Sowohl die Dioden 3 als auch die Magnetspule l können entweder kontinuierlich oder in einer bestimmten zeitlichen Abfolge gepulst betrieben werden.

Als Infrarotemitter 2 können auch Infrarotlampen eingesetzt werden.

## Ansprüche

l. Bioenergetisches Behandlungsgerät zur Erzeugung elektromagnetischer Felder und Wellen, durch welche bioenergetische Wirkungen im menschlichen oder tierischen Organismus hervorgerufen werden, mit einer Magnetfeldspule (l) in einem Gehäuse bzw. einer Halterung, dadurch gekennzeichnet, daß im Inneren der Spule (l) oder an hrem Gehäuse mindestens ein Infrarotemitter (2) angebracht ist.

2. Behandlungsgerät nach Anspruch l, dadurch gekennzeichnet, daß der Infrarotemitter (2) mindestens aus einer Infrarotstrahlung emittierenden Diode (3) besteht.

3. Behandlungsgerät nach Anspruch l oder 2, dadurch gekennzeichnet, daß die Infrarotemitter - (2) in einer Querschnittsebene einer Spule (l) angeordnet sind.

4. Behandlungsgerät nach Anspruch 3, dadurch gekennzeichnet, daß drei Spulen (l) mit Infrarotemittern (2) nebeneinander angeordnet sind, wobei die Querschnittsebene der beiden äußeren Spulen (l) mit der der mittleren Spule (l) einen Winkel von ca. l20° einschließen.

5. Behandlungsgerät nach Anspruch l oder 2, dadurch gekennzeichnet, daß Infrarotemitter (2) auf einer zur Spulenachse konzentrischen Zylinderfläche angeordnet sind.

6. Behandlungsgerät nach einem der Ansprüche l bis 4, dadurch gekennzeichnet, daß das Infrarotlicht durch einen Spiegel abgelenkt und/oder gebündelt wird.

Fig.1

Fig.2